# EUROPÄISCHE PATENTSCHRIFT

(11) **EP 1 099 431 B1**
(45) Veröffentlichungstag und Bekanntmachung des Hinweises auf die Patenterteilung: **03.05.2006**
(21) Anmeldenummer: 00120807.3
(22) Anmeldetag: 23.09.2000
(51) Int. Cl.: A61F 2/60, A61F 2/68

(54) **Beinprothese mit verschwenkbarem Prothesenfuss**
Leg prosthesis with pivotable prosthetic foot
Prothèse de jambe avec un pied prothétique pivotable

(30) Priorität: 10.11.1999 DE 19953972
(43) Veröffentlichungstag der Anmeldung: 16.05.2001
(73) Patentinhaber: SCHÜTT & GRUNDEI ORTHOPÄDIETECHNIK GmbH, D-23556 Lübeck (DE)
(72) Erfinder: Grundei, Hans, Dr., 23556 Lübeck (DE)
(74) Vertreter: Fuchs Mehler Weiss & Fritzsche

(56) Entgegenhaltungen:
- EP-A- 0 358 056
- DE-C- 19 845 191
- US-A- 2 568 051
- US-A- 2 568 053
- US-A- 3 947 897
- US-A- 5 509 936

## Beschreibung

Die vorliegende Erfindung betrifft eine Beinprothese zur Adaption an einen Oberschenkelstumpf. Diese besteht aus einem Adapter für ein Kniegelenk, wie er beispielsweise bekannt ist aus der US-A-3,947,897. Dieser wird mit einem Stielteil im Femurstumpf des Patienten verankert. Distal tritt der Adapter aus dem Oberschenkelstumpf aus und bietet dort eine Ankopplungsmöglichkeit für ein künstliches Kniegelenk, wie es beispielsweise bekannt ist aus der EP-B-0 358 056.

Üblicherweise ist an das Kniegelenk ein Prothesenunterschenkel angekoppelt, welcher seinerseits distal einen angelenkten Prothesenfuß trägt. Dieser läßt sich in eine Spitzfußstellung schwenken.

Das Kniegelenk ist gemäß dem Vorschlag beispielsweise der EP-B-0 358 056 so ausgebildet, daß es beim Übergang von der Strecklage in die Beugelage um eine Schwenkachse eine kombinierte Roll- und Gleitbewegung ausführt. Im Gegensatz zu einem reinen Scharniergelenk ist das Kniegelenk der gattungsgemäßen Beinprothese so ausgebildet, daß sich der Abstand eines dorsal gesehen vor der Schwenkachse gelegenen Punktes des Kniegelenks zum Ende des Prothesenunterschenkels stetig verkleinert. Mit anderen Worten vergrößert sich der Abstand eines dagegen ventral gesehen vor der Schwenkachse gelegenen Punktes zum Ende des Prothesenunterschenkels stetig.

Ein Problem für den Patienten mit teilamputiertem Oberschenkel ist neben anderen, das er beim Gehen mit dem gesunden Fuß in eine stärkere Spitzfußstellung gehen muß, um den Prothesenfuß beim erneuten Schritt nach vorn mit der Beinprothese durchpendeln zu lassen. Dies gilt unabhängig davon, ob der Prothesenfuß an dem Prothesenunterschenkel nun verschwenkbar oder aber fest arretiert ist. Die Notwendigkeit, den gesunden natürlichen Fuß in eine verstärkte oder extreme Spitzfußstellung zu bringen, damit die Prothese durchpendeln kann, bedingt eine recht unphysiologische Bewegungsweise und damit einhergehend eine starke Beanspruchung der Wirbelsäule.

Vor diesem Hintergrund ist es die Aufgabe der vorliegenden Erfindung, hier Abhilfe zu schaffen, d. h. eine gattungsgemäße Beinprothese so weiterzubilden, daß die Notwendigkeit, den gesunden natürlichen Fuß in eine unphysiologische Spitzfußstellung zu bringen, damit das künstliche Bein durchpendeln kann, nicht mehr gegeben ist und daß der Bewegungsablauf natürlicher erscheint.

Gelöst wird diese Aufgabe dadurch, daß zwischen wenigstens einem von dorsal gesehen vor der Schwenkachse gelegenen Lagerpunkt und/oder einen ventral gesehen vor der Schwenkachse gelegenen Lagerpunkt und dem Prothesenfuß ein Kraftübertragungselement angeordnet ist, welches den Prothesenfuß beim Beugen des Kniegelenkes aus einer Spitzfußlage oder Mittelfußstellung des Kunstfusses in zunehmenden Maße in die Hackenfußlage überführt.

Vorliegend wird also durch die Bewegung des Knies gesteuert aktiv eine Stellkraft in den Prothesenfuß eingeleitet, derart, daß mit zunehmender Beugung des Knies der Prothesenfuß in einem zunehmenden Maße in die Hackenfußlage gebracht wird. Hierdurch verkürzt sich der notwendige Hub für den gesunden Fuß, damit die Beinprothese durchpendeln kann, leicht um 5 bis 10 mm und damit in einem Maße, welches die Bewegung des Patienten natürlicher erscheinen läßt. Extreme Ausgleichsbewegungen sind so nicht mehr notwendig.

Auf die Bedingung, daß sich der Abstand eines dorsal gesehen vor der Schwenkachse gelegenen Punktes zum Ende des Prothesenunterschenkels stetig verkleinert, wird besonders hingewiesen. Bei einem Kniegelenk mit einem reinen Scharniergelenk wäre diese Bedingung beispielsweise nicht gegeben.
Die dann vorgegebene Polkurve würde zwar beispielsweise bei der ersten Bewegung von der Strecklage hin zur Beugelage ebenfalls eine Verringerung des Abstandes mit sich bringen. Nach Erreichen eines Totpunktes aber würde sich der Abstand wieder vergrößern. Dies würde bei einer Beinprothese bei Durchführung der kompletten Bewegung des Kniegelenkes von der Strecklage zur Beugelage dazu führen, daß der Fuß zunächst leicht in die Hackenfußlage geschwenkt wird, nach Überschreiten des besagten Totpunktes aber wieder in die Ausgangslage oder Spitzfußstellung geschwenkt würde, so daß im Ergebnis bei voller Beugung des Kniegelenkes wieder eine quasi Spitzfußstellung des Fußes gegenüber des Prothesenunterschenkels vorläge. Genau dieses Phänomen gilt es aber zu vermeiden, um eben dann die Notwendigkeit, den gesunden Fuß verstärkt in eine Spitzfußstellung zu bringen, damit die Beinprothese durchpendeln kann, nicht mehr vorliegt.

Gemäß einer bevorzugten Ausführungsform ist es vorgesehen, daß das Kraftübertragungselement aus einer am Kniegelenk und an dem Prothesenfuß angelenkten Schubstange aufgebaut ist. Am proximalen Ende kann die Schubstange beispielsweise im dorsalen Bereich des Kniegelenkes angelenkt sein, um die sich bei der Verringerung des Abstandes dieses Anlenkpunktes zum Ende des Prothesenunterschenkels für die Ausführung der Schwenkbewegung des Prothesenfußes von der Ausgangslage hin in die gewünschte Hackenfußlage auszunutzen.

Besonders zuverlässig wird der Prothesenfuß in die Hackenfußlage bei der Bewegung des Kniegelenks von der Streck- in die Beugelage bewegt, wenn die Beinprothese gemäß einer vorteilhaften Weiterbildung so ausgebildet ist, daß der Prothesenfuß an den Prothesenunterschenkel um einen ventral gelegenen Schwenkpunkt schwenkbar gekoppelt ist und daß das Kraftübertragungselement an den Prothesenfuß an einem dorsal gelegenen Lagerpunkt angelenkt ist. Das Kraftübertragungselement bewirkt dann die Einleitung eines Drehmomentes, um den dorsal gelegenen Lagerpunkt herum, wodurch der Prothesenfuß sicher in die Hackenfußlage schwenkt.

Gemäß einer besonders bevorzugten Ausführungsform wird vorgesehen, daß zwischen dem Prothesenfuß und einem Lager an dem Prothesenunterschenkel ein flexibler, auf seine effektive Länge einstellbarer Zügel gespannt ist, der bei zunehmender Beugung des Kniegelenkes eine zunehmende Lose erhält.

Der Zügel übernimmt im wesentlichen die Aufgabe der natürlichen Achillesferse. Die Hauptaufgabe des Zügels besteht darin, in Streckstellung des Kniegelenkes den Fuß in seine Ausgangslage zurückzubringen. Des weiteren dient der Zügel aufgrund der Einstellmöglichkeit seiner effektiven Länge beispielsweise durch einen Anschlag mit einem Gewinde, mit welchem das betreffende Ende des Zügels verschraubbar ist, zur individuellen Einstellung einer Spitzfußstellung des Prothesenfußes. Diese Einstellung differiert in der Regel durch die unterschiedlichen Fußabsätze von Patient zu Patient.

In dem Kraftübertragungselement ist vorzugsweise ein Rückstellelement integriert, welches bei Streckung des Knies nach vorheriger Beugung den Prothesenfuß aktiv wieder in seine Ausgangslage verbringt. Dieses aktive Rückstellelement unterstützt die Wirkung des weiter oben erwähnten Zügels bei beginnender Bewegung von der Beugelage in die Strecklage des Knies. Dabei entfaltet in erster Linie das erwähnte Kraftübertragungselement die Rückstellwirkung, wohingegen bei annäherndem Erreichen der Strecklage der Zügel in erster Linie seine Wirkung entfaltet.

Bei der vorerwähnten Weiterbildung ist es bevorzugt, daß das Rückstellelement eine eine Spiralfeder aufnehmende Führungshülse sowie einen in der Hülse geführten Stempel als Teil der Schubstange aufweist, derart, daß bei zunehmender Beugung des Kniegelenkes die Feder zunehmend druckbelastet wird und bei Streckung des Kniegelenkes die Federkraft den Prothesenfuß in die Ausgangsposition verschwenkt. Je stärker also das Knie gebeugt wird, desto höhere Federkräfte werden in dem Rückstellelement erzeugt.

Schließlich wird vorteilhaft vorgesehen, daß die Führungshülse in einem an dem Prothesenunterschenkel befestigten Gehäuse gelagert ist. Hierdurch ergibt sich eine kompakte Einheit, die für den Patienten relativ einheitlich zu handhaben ist.

Die Erfindung wird gemäß eines Ausführungsbeispiels gemäß der Zeichnungsfiguren näher erläutert. Hierbei zeigt:

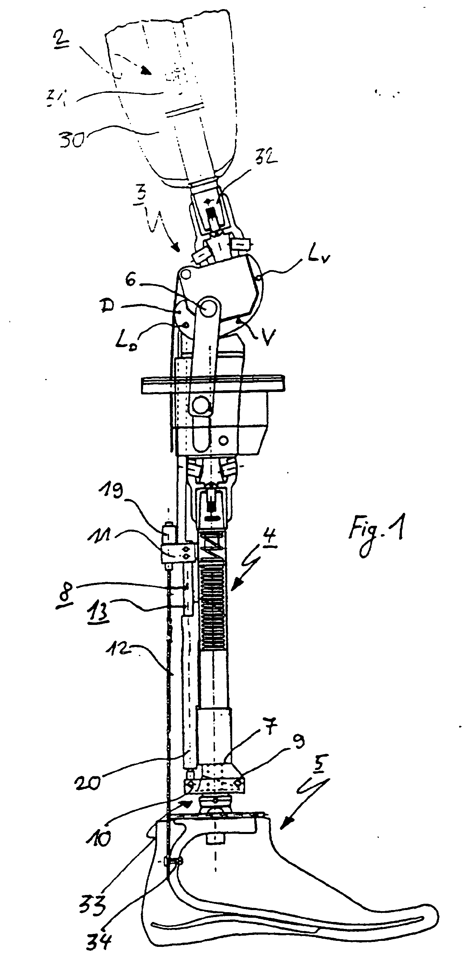

- Fig. 1: die schematische Seitenansicht der vollständigen Beinprothese, und
- Fig. 2: eine Detaildarstellung aus Fig. 1, teilweise geschnitten.

Nachfolgend bezeichnen gleiche Bezugszeichen dieselben Teile.

Die Beinprothese besteht aus einem Adapter 2, der mit dem Oberschenkelstumpf 30 des Patienten in der Weise verbunden ist, daß der Adapter 2 an dem Femurstumpf 31 fixiert ist. An das Ankoppelungselement 32 des Adapters 2 ist ein Kniegelenk 3 gekoppelt, dem sich distal ein Prothesenunterschenkel 4 anschließt, welcher schließlich einen Prothesenfuß 5 trägt, in der Weise, daß der Prothesenfuß 5 mit dem Prothesenunterschenkel 4 schwenkbar verbunden ist.

Das Kniegelenk 3 weist eine Schwenkachse 6 auf, um welche das Oberschenkelteil des Kniegelenks 3 gegenüber dem Unterschenkelteil 4 schwenkbar ist. Das Kniegelenk 3 hat die spezielle Eigenschaft, daß es bei dem Übergang von der Strecklage hin zur Beugelage um die Schwenkachse 6 eine Roll-Gleitbewegung ausführt. Diese führt dazu, daß sich der Abstand eines dorsal gesehen vor der Schwenkachse 6 gelegenen Punktes D zum Ende 7 des Prothesenunterschenkels 4 stetig verkleinert. Entsprechend vergrößert sich der besagte Abstand bei einem ventral gesehen vor der Schwenkachse 6 gelegenen Punktes V stetig bei Ausführung der Bewegung von der Streck- in die Beugelage des Kniegelenkes 3.

Der Prothesenfuß 5 ist am Ende 7 des Prothesenunterschenkels 4 um einen ventral gelegenen Schwenkpunkt 9 schwenkbar angelenkt. Wird der Prothesenfuß 5 um den Schwenkpunkt 9 geschwenkt, begibt sich der Prothesenfuß 5 in die Hackenfußlage.

Die Koppelung der Bewegung des Kniegelenks 3 mit der Bewegung dem Prothesenfuß 5 gelingt über das Kraftübertragungselement 8, welches proximal an einem Lagerpunkt L_{D} am Kniegelenk 3 angelenkt ist. Der Lagerpunkt L_{D} liegt dorsal gesehen vor der Schwenkachse 6. Alternativ oder zusätzlich könnte ein Kraftübertragungselement auch am ventral gesehen vor der Schwenkachse 6 gelegenen Lagerpunkt L_{V} angelenkt sein. Dann allerdings müßte beispielsweise über einen Rollenmechanismus die Kraft entsprechend umgelenkt werden, damit die für die Verschwenkung des Prothesenfußes 5 erforderliche Kraft von dorsal so eingeleitet werden könnte, daß die Verschwenkbewegung um den Schwenkpunkt 9 ausgeführt werden kann. Die Kraft des Kraftübertragungselementes 8 wird in das Ansatzstück 33 am Lagerpunkt 10 dorsal eingeleitet. Das Ansatzstück 33 übernimmt hierbei die Funktion des natürlichen oberen Sprunggelenkes.

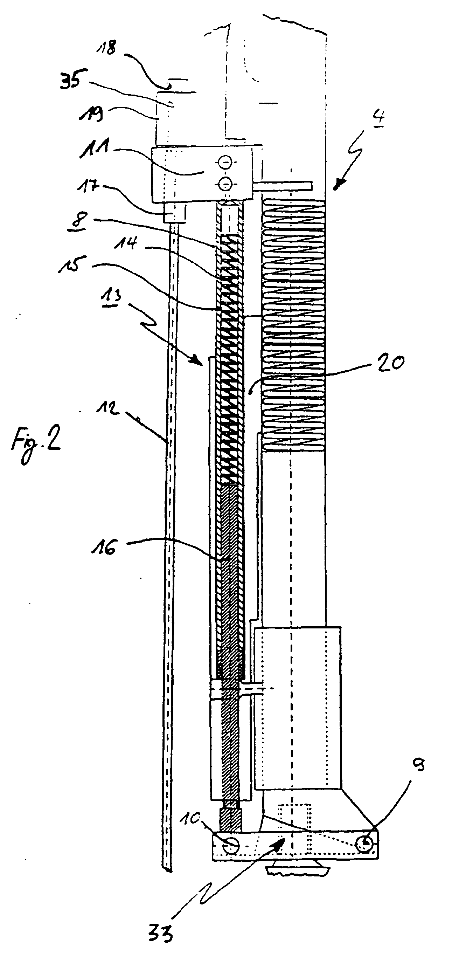

Darüber hinaus ist bei dem gezeigten Ausführungsbeispiel dorsal zwischen einem an dem Prothesenunterschenkel 4 fixierten Lager 11 und dem Prothesenfuß 5, hier am Lagerpunkt 34 ein flexibler Zügel 12 gespannt. Der Zügel 12 besteht beispielsweise aus einer flexiblen Stahllitze. Er übernimmt die Funktion der natürlichen Achillessehne. Er dient dazu, beim Übergang von der Beuge- in die Strecklage des Kniegelenkes, den Prothesenfuß 5 wieder vollständig in die Ausgangslage zurückzuschwenken. Darüber hinaus dient er der individuellen Einstellung einer Hackenlage des Prothesenfußes 5. Dazu weist der Zügel 12 am proximalen Ende eine Gewindehülse 17 auf, welche mit einem Ansatz 19 mit Innengewinde sowie dem Lager 11 zusammenarbeitet, derart, daß der Ansatz 19 einen Anschlag am Lager 11 bildet. Durch Verschraubung des Ansatzes 19 auf dem Gewinde 35 (Fig. 2) läßt sich patientenindividuell die Ausgangsfußstellung (Spitzfußstellung) einstellen.

Weitere Einzelheiten sind in Fig. 2 dargestellt.

Hier ergibt sich noch einmal, wie auf dem proximalen Ende des Zügels 12 eine Hülse 17 gesetzt ist mit einem Gewinde 18, welches mit dem Ansatz 19 und dessen Innengewinde 35 zusammenarbeitet.

Der Ansatz 19 bildet hier ein Anschlagelement an dem mit dem Prothesenunterschenkel 4 verbundenen Lager 11.

Vorliegend ist in dem Kraftübertragungselement 8 ein Rückstellelement 13 integriert, welches vorliegend eine Führungshülse 15 umfaßt, in deren Inneren eine Spiralfeder 14 angeordnet ist. Die Führungshülse 15 mit der Spiralfeder 14 arbeitet mit einem im Inneren der Hülse 15 geführten Stempel 16 zusammen, derart, daß bei erhöhter Kraftbeaufschlagung der Hülse 15 in Fig. 2 von oben her bei verstärkter Beugebewegung des Kniegelenks 3 die Feder 14 im Inneren der Hülse 15 zunehmend druckbelastet wird. Als Reaktion hierauf leitet der Stempel 16 eine entsprechende Kraft in das Ansatzstück 33 weiter, und zwar in den Lagerpunkt 10, woraufhin der Prothesenfuß 5 zu einer Schwenkbewegung um den Lagerpunkt 9 veranlaßt wird.

Wesentlich bei der erfindungsgemäßen Beinprothese ist vor allem, daß vorliegend eine Zwangskoppelung zwischen der Bewegung des Kniegelenks 3 und der Verschwenkbewegung des Prothesenfußes 5 realisiert ist. Voraussetzung für die stetig zunehmende Verschwenkung des Prothesenfußes mit zunehmender Beugelage des Kniegelenkes ist die bereits erwähnte Ausbildung des Kniegelenkes, das eine Roll-/Gleitbewegung ausführen muß. Bei einem reinen Scharniergelenk als Kniegelenk ist es aufgrund der Geometrie nicht möglich, die gewünschte Schwenkbewegung des Prothesenfußes zu erzielen.

## Patentansprüche

1. Beinprothese zur Adaption an einen Oberschenkelstumpf, bestehend aus einem Adapter (2) für ein Kniegelenk (3), einem daran befestigten Kniegelenk (3) und einem am Kniegelenk (3) angekoppelten Prothesenunterschenkel (4) mit einem daran angelenkten Prothesenfuß (5), der in eine Hackenfußlage schwenkbar ist, wobei das Kniegelenk (3) so ausgebildet ist, daß es beim Übergang von der Strecklage in die Beugelage um eine Schwenkachse (6) eine kombinierte Roll-Gleitbewegung ausführt, derart, daß sich der Abstand eines dorsal gesehen vor der Schwenkachse (6) gelegenen Punktes (D) zum Ende (7) des Prothesenunterschenkels (4) stetig verkleinert bzw. eines ventral gesehen vor der Schwenkachse (6) gelegenen Punktes (V) zum Ende (7) des Prothesenunterschenkels (4) stetig vergrößert,
**dadurch gekennzeichnet,**
**daß** zwischen wenigstens einem von dorsal gesehen vor der Schwenkachse (6) gelegener Lagerpunkt (L_{D}) und/oder einem ventral gesehen vor der Schwenkachse (6) gelegenen Lagerpunkt (L_{V}) und dem Prothesenfuß (5) ein Kraftübertragungselement (8) angeordnet ist, welches den Prothesenfuß (5) beim Beugen des Kniegelenkes (3) in zunehmendem Maße aus der Spitzfußlage in die Hackenfußlage überführt.

2. Beinprothese nach Anspruch 1, **dadurch gekennzeichnet, daß** das Kraftübertragungselement (8) aus einer am Kniegelenk (3) und an dem Prothesefuß (5) angelenkten Schubstange aufgebaut ist.

3. Beinprothese nach Anspruch 2 oder 3, **dadurch gekennzeichnet, daß** der Prothesenfuß (5) an dem Prothesenunterschenkel (4) um einen ventral gelegenen Schwenkpunkt (9) schwenkbar gekoppelt ist und daß das Kraftübertragungselement (8) an den Prothesenfuß (5) an einem dorsal gelegenen Lagerpunkt (10) angelenkt ist.

4. Beinprothese nach einem der Ansprüche 1 bis 3, **dadurch gekennzeichnet, daß** zwischen dem Prothesenfuß (5) und einem Lager (11) an dem Prothesenunterschenkel (4) ein flexibler, auf seine effektive Länge einstellbarer Zügel (12) gespannt ist, der bei zunehmender Beugung des Kniegelenkes (3) eine zunehmende Lose erhält.

5. Beinprothese nach einem der Ansprüche 1 bis 4, **dadurch gekennzeichnet, daß** in dem Kraftübertragungselement (8) ein Rückstellelement (13) integriert ist, welches bei Streckung des Kniegelenkes (3) nach vorheriger Beugung den Prothesenfuß (5) wieder in seine Ausgangslage (Spitzfußlage) verbringt.

6. Beinprothese nach Anspruch 5, **dadurch gekennzeichnet, daß** das Rückstellelement (13) eine eine Spiralfeder (14) aufnehmende Führungshülse (15) sowie einen in der Hülse (15) geführten Stempel (16) als Teil der Schubstange aufweist, derart, daß bei zunehmender Beugung des Kniegelenks (3) die Feder (14) zunehmend druckbelastet wird und bei Streckung des Kniegelenkes (3) die Federkraft den Prothesenfuß (5) in die Ausgangsposition verschwenkt.

7. Beinprothese nach Anspruch 6, **dadurch gekennzeichnet, daß** die Führungshülse (15) in einem an den Prothesenunterschenkel (4) befestigten Gehäuse (20) gelagert ist.

## Claims

1. Leg prosthesis for adaptation to a thigh stump consisting of an adapter (2) for a knee joint (3), a knee joint (3) attached thereto and a prosthesis lower leg (4) coupled to the knee joint (3) having a prosthesis foot (5) hinged thereto, which can be pivoted into a club foot position, wherein the knee joint (3) is designed so that it executes a combined rolling and gliding movement during transition from the extended position into the bent position about a pivoting axis (6), such that the distance of a point (D) placed in front of the pivoting axis (6) seen dorsally, to the end (7) of the prosthesis lower leg (4) is reduced continuously or a point (V) placed in front of the pivoting axis (6) seen ventrally, to the end (7) of the prosthesis lower leg (4) is increased continuously, **characterised in that** a force-transmitting element (8), which transfers the prosthesis foot (5) into the club foot position when bending the knee joint (3) to increasing extent from the pointed foot position, is arranged between at least one bearing point (L_{D}) placed in front of the pivoting axis (6) seen dorsally, and/or a bearing point (L_{V}) placed in front of the pivoting axis (6) seen ventrally, and the prosthesis foot (5).

2. Leg prosthesis according to claim 1, **characterised in that** the force-transmitting element (8) is constructed from a connecting rod hinged to the knee joint (3) and to the prosthesis foot (5).

3. Leg prosthesis according to claim 2 or 3, **characterised in that** the prosthesis foot (5) is coupled pivotably to the prosthesis lower leg (4) about a ventrally placed pivoting point (9) and **in that** the force-transmitting element (8) is hinged to the prosthesis foot (5) at a dorsally placed bearing point (10).

4. Leg prosthesis according to one of claims 1 to 3, **characterised in that** a flexible restraint (12), which can be adjusted over its effective length and which has increasing slackness during increasing bending of the knee joint (3), is clamped between the prosthesis foot (5) and a bearing (11) on the prosthesis lower leg (4).

5. Leg prosthesis according to one of claims 1 to 4, **characterised in that** a restoring element (13), which brings the prosthesis foot (5) back to its starting position (pointed foot position) during extension of the knee joint (3) after prior bending, is integrated into the force-transmitting element (8).

6. Leg prosthesis according to claim 5, **characterised in that** the restoring element (13) has a guide sleeve (15) accommodating a helical spring (14) and a ram (16) guided in the sleeve (15) as part of the connecting rod, such that the spring (14) is compressively stressed increasingly during increasing bending of the knee joint (3) and the spring force pivots the prosthesis foot (5) into the starting position during extension of the knee joint (3).

7. Leg prosthesis according to claim 6, **characterised in that** the guide sleeve (15) is mounted in a housing (20) attached to the prosthesis lower leg (4).

## Revendications

1. Prothèse de jambe destinée à être adaptée à un moignon de cuisse, constituée d'un adaptateur (2) pour une articulation du genou (3), d'une articulation du genou (3) fixée sur celui-ci et d'une partie inférieure de jambe prothétique (4) accouplée à l'articulation du genou (3), avec un pied prothétique (5) articulé à celle-ci, qui peut pivoter dans une position fléchie du pied, l'articulation du genou (3) étant réalisée de telle sorte qu'elle exécute lors du passage de la position tendue dans la position fléchie un mouvement de roulement-glissement combiné autour d'un axe de pivotement (6), de telle sorte que la distance d'un point (D) placé avant l'axe de pivotement (6) vu dans la direction dorsale à l'extrémité (7) de la partie inférieure de jambe prothétique (4) soit constamment réduite ou que la distance d'un point (V) placé avant l'axe de pivotement (6) vu dans la direction ventrale à l'extrémité (7) de la partie inférieure de jambe prothétique (4) soit constamment augmentée,
**caractérisée en ce**
**qu'**entre au moins un point d'appui (L_{D}) placé avant l'axe de pivotement (6) vu dans la direction dorsale et/ou un point d'appui (L_{V}) placé avant l'axe de pivotement (6) vu dans la direction ventrale, et le pied prothétique (5), on dispose un élément de transfert de force (8) qui fait passer le pied prothétique (5) lors du fléchissement de l'articulation du genou (3) de manière croissante de la position tendue du pied dans la position fléchie du pied.

2. Prothèse de jambe selon la revendication 1, **caractérisée en ce que** l'élément de transfert de force (8) est constitué d'une tringle de poussée articulée à l'articulation du genou (3) et au pied prothétique (5).

3. Prothèse de jambe selon la revendication 2 ou 3, **caractérisée en ce que** le pied prothétique (5) est accouplé à la partie inférieure de jambe prothétique (4) de manière à pouvoir pivoter autour d'un pivot (9) situé en position ventrale, et **en ce que** l'élément de transfert de force (8) est articulé au pied prothétique (5) au niveau d'un pivot (10) situé en position dorsale.

4. Prothèse de jambe selon l'une quelconque des revendications 1 à 3, **caractérisée en ce qu'**entre le pied prothétique (5) et un palier (11) sur la partie inférieure de jambe prothétique (4) est tendue une bride flexible (12) ajustable à sa longueur efficace, qui acquiert un relâchement croissant au fur et à mesure du fléchissement de l'articulation du genou (3).

5. Prothèse de jambe selon l'une quelconque des revendications 1 à 4, **caractérisée en ce que** dans l'élément de transfert de force (8) est intégré un élément de rappel (13), qui lors de l'étirement de l'articulation du genou (3), après flexion préalable, ramène le pied prothétique (5) à nouveau dans sa position de départ (position tendue).

6. Prothèse de jambe selon la revendication 5, **caractérisée en ce que** l'élément de rappel (13) présente une gaine de guidage (15) recevant un ressort spiral (14) ainsi qu'un poinçon (16) guidé dans la gaine (15) et faisant partie de la tringle de poussée, de telle sorte qu'au fur et à mesure du fléchissement de l'articulation du genou (3), le ressort (14) soit de plus en plus sollicité en pression et que lors de l'étirement de l'articulation du genou (3), la force du ressort fasse pivoter le pied prothétique (5) dans la position de départ.

7. Prothèse de jambe selon la revendication 6, **caractérisée en ce que** la gaine de guidage (15) est montée dans un boîtier (20) fixé à la partie inférieure de jambe prothétique (4).
